# EUROPÄISCHE PATENTANMELDUNG

(11) **EP 3 854 358 A1**
(43) Veröffentlichungstag der Anmeldung: **28.07.2021**
(21) Anmeldenummer: 21150861.9
(22) Anmeldetag: 11.01.2021
(51) Int. Cl.: A61F 5/01, A61F 5/30

(54) **KNIEGELENKBANDAGE**

(30) Priorität: 24.01.2020 DE 102020101744
(71) Anmelder: Julius Zorn GmbH, 86551 Aichach (DE)
(72) Erfinder: Fischer, Markus, 80687 München (DE); Berg, Roland, 12527 Berlin (DE)
(74) Vertreter: Charrier Rapp & Liebau

(57) **Zusammenfassung**

Die Erfindung betrifft eine Bandage für ein Kniegelenk (K), umfassend einen schlauchförmigen Grundkörper (1) aus einem elastischen Textilmaterial mit einer bei am Kniegelenk (G) angelegter Bandage dem Kniegelenk zugewandten Innenseite (i), zumindest eine am Grundkörper angeordnete erste Pelotte (2), welche bei angelegter Bandage proximal zur Kniescheibe positioniert ist und die Kniescheibe zumindest teilweise umgreift, sowie eine distal zur ersten Pelotte angeordnete zweite Pelotte (6), welche bei angelegter Bandage über die Patellasehne (P) verläuft. Die zweite Pelotte ist dabei abnehmbar an der Innenseite des Grundkörpers befestigt. Dadurch kann die Bandage für unterschiedliche Therapiephasen sowohl mit als auch ohne die zweite Pelotte verwendet werden und die zweite Pelotte kann auch separat in einer Patellasehnenbandage eingesetzt werden.

## Beschreibung

Die Erfindung betrifft eine Kniegelenkbandage nach dem Oberbegriff des Anspruchs 1.

Aus dem Stand der Technik sind Orthesen in Form von textilen Bandagen zur Korrektur und/oder Entlastung des Kniegelenks und insbesondere zur Beeinflussung des Patellagleitwegs bekannt. Diese Orthesen umfassen einen schlauchförmigen Grundkörper aus einem Textilmaterial und weisen mindestens eine, lokal um die Patella angeordnete Druckpelotte aus einem flexiblen Material, beispielsweise aus Silikon, auf. Die wenigstens eine Pelotte gibt der Patella bei Bewegungen des Kniegelenks eine Führung und kann dadurch bei Schädigung des Kniegelenks im Gleitlager zwischen der Patella und dem Oberschenkel zu einer Schmerzreduktion beitragen. Durch einstellbare Zug- oder Druckelemente, wie z.B. Zugbänder, die mit der Pelotte zugfest verbunden sind, soll eine positive Beeinflussung des Patellagleitwegs bei einer Bewegung des Kniegelenks in einer physiologisch günstigen Position erreicht werden.

Weiterhin sind Patellasehnenbandagen bekannt, welche eine quer über die Patellasehne verlaufenden Pelotte aufweisen, die zu einer Vorspannung der Patellasehne sowie zu einer Stabilisierung des Kniegelenks und dadurch zu einer Schmerzlinderung beiträgt. Eine solche Patellasehnenbandage ist bspw. aus der EP 3086746 B1 bekannt, in der eine Patellasehnenbandage mit einem Bandagenelement und mehreren daran nebeneinander angeordneten Pelottenkörpern zum Aufbau eines infrapatellaren Drucks und zum Einwirken auf im seitlichen Bereich des Kniegelenks verlaufende Muskel- und Sehnengruppen beschrieben ist. Das Bandagenelement umfasst dabei einen längenverstellbaren Gurt, mit dem die Patellasehnenbandage am Kniegelenk unterhalb der Patella und quer über die Patellasehne verlaufend angelegt werden kann.

Bei Verwendung solcher Patellasehnenbandagen kann es bei einer Bewegung des Kniegelenks zu einem Hochschieben der Patella in eine physiologisch ungünstige Position kommen.

Um dies zu verhindern schlägt die EP 2 954 879 B1 eine Kniegelenkbandage mit einem schlauchförmigen elastischen Gestrickkörper vor, die eine erste Pelotte und eine zweite Pelotte aufweist, wobei die erste Pelotte quer über die Patellasehne verläuft, um durch gezielt auf die Patellasehne ausgeübten Druck durch Entzündungen der Patellasehne hervorgerufene Schmerzen zu lindern, wie sie beispielsweise beim Patellasehnenspitzensyndrom auftreten können, und die zweite Pelotte höhenmäßig versetzt zu ersten Pelotte angeordnet und in der Tragstellung oberhalb der Patella positioniert ist. Die erste Pelotte ist dabei in einer am Gestrickkörper ausgebildeten Tasche angeordnet. Oberhalb der ersten Pelotte verläuft dabei abschnittsweise um den schlauchförmigen Gestrickkörper ein Spanngurt, mit dem der von der ersten Pelotte auf die Patellasehne ausgeübte Druck eingestellt werden kann. Die hufeisenförmig ausgebildete zweite Pelotte dient dazu, ein Hochschieben der Patella, resultierend aus dem von der ersten Pelotte auf die Patellasehne ausgeübten Druck, zu verhindern. Die zweite Pelotte stellt dabei allerdings nur ein passives Element dar, das bei einer Bewegung des Kniegelenks eine passive Führung für die Patella bereitstellt. Weiterhin kann diese bekannte Kniegelenkbandage nur zur Therapie bestimmter Kniegelenksbeschwerden wie dem Patellaspitzensyndrom eingesetzt werden.

Hiervon ausgehend besteht ein Bedürfnis für eine Kniegelenkbandage, die einerseits eine aktive Stabilisierung der Patella, insbesondere in lateraler Richtung, bewirkt und andererseits einem Hochschieben der Patella bei einer Bewegung des Kniegelenks entgegenwirkt und darüber hinaus in möglichst flexibler Weise und in unterschiedlichen Therapieformen und -stufen einen gezielten Druck auf die Patellasehne ausüben kann, um Patellasehnenreizungen zu lindern.

Der Erfindung liegt daher die Aufgabe zugrunde, eine Kniegelenkbandage bereit zu stellen, die bei einer Therapie von Kniegelenksbeschwerden eine optimierte Bewegungsführung des Kniegelenks ermöglicht und insbesondere einerseits ein Abdriften der Patella nach außen (also in lateraler Richtung) und andererseits ein Hochschieben der Patella in proximaler Richtung unterdrückt und gleichzeitig durch gezielten Druck auf die Patellasehne Patellasehnenreizungen lindern kann. Dabei soll die Kniegelenkbandage eine Therapie von unterschiedlichen Kniegelenksbeschwerden in möglichst flexibler Weise, insbesondere in unterschiedlichen Therapiestufen, das Kniegelenk bei einer Bewegung in einer physiologisch optimierten Position halten und dabei eine individuelle Anpassung an die Anatomie eines Patienten ermöglichen. Weiterhin soll durch die Kniegelenkbandage eine Durchblutung des das Kniegelenk umgebenden Gewebes gefördert, Schwellungen und Ergüsse abgebaut sowie Reizungen und Schmerzen gelindert werden.

Diese Aufgaben werden mit einer Kniegelenkbandage mit den Merkmalen des Anspruchs 1 sowie mit einem Bandagenset nach Anspruch 11 gelöst. Bevorzugte Ausführungsformen dieser Kniegelenkbandage und des Bandagensets sind den abhängigen Ansprüchen zu entnehmen.

Die Kniegelenkbandage gemäß der Erfindung umfasst einen schlauchförmigen Grundkörper aus einem elastischen Textilmaterial mit einer bei am Kniegelenk angelegter Bandage dem Kniegelenk zugewandten Innenseite, zumindest eine am Grundkörper angeordnete erste Pelotte, welche bei angelegter Bandage proximal zur Patella positioniert ist und die Patella zumindest teilweise umgreift, sowie eine distal zur ersten Pelotte angeordnete zweite Pelotte, welche bei angelegter Bandage über die Patellasehne verläuft, wobei die zweite Pelotte abnehmbar an der Innenseite des Grundkörpers befestigt ist.

Dadurch kann die Kniegelenkbandage gemäß der Erfindung in flexibler Weise sowohl mit als auch ohne die zweite Pelotte eingesetzt werden, indem die zweite Pelotte entweder mittels lösbarer Befestigungsmittel an der Innenseite des Grundkörpers befestigt und die Bandage in dieser Form an ein zu therapierendes Kniegelenk angelegt wird, oder indem die zweite Pelotte durch Lösen der Befestigungsmittel von dem Grundkörper abgenommen und dieser nur mit der zweckmäßig darin integrierten ersten Pelotte an ein Kniegelenk angelegt wird. Weiterhin kann die vom Grundkörper abgenommene zweite Pelotte auch in Verbindung mit einem zugehörigen Halteband als Patellasehnenbandage ohne den Grundkörper und die darin integrierte erste Pelotte, eingesetzt werden, indem die vom Grundkörper abgenommene zweite Pelotte mittles lösbarer Befestigungsmittel an ein Halteband befestigt und mit diesem Halteband an einem Kniegelenk unterhalb der Patella angelegt wird.

Die zweite Pelotte übt sowohl in der Funktionsstellung bei angelegter Kniegelenkbandage mit an der Innenseite des Grundkörpers befestigter zweiter Pelotte als auch bei Anlegung der zweiten Pelotte am Kniegelenk mit dem Halteband als Patellasehnenbandage jeweils einen dosierten Druck auf die Patellasehne aus, insbesondere an ihrem Ansatz, und kann dadurch die Patellasehne entlasten. Weiterhin führt die zweite Pelotte zu einer Vorspannung der Patellasehne, wodurch bei einer Bewegung des Kniegelenks eine weitere Stabilisierung erfolgt.

Durch die zweite Pelotte können daher Patellasehnenreizungen therapiert und dadurch hervorgerufene Schmerzen gelindert werden.

Bevorzugt ist die zweite Pelotte mittles lösbarer Befestigungsmittel, wie z.B. Klettverschlüsse, abnehmbar an der dem Kniegelenk zugewandten Innenseite des Grundkörpers befestigbar. Durch die lösbare Anordnung der zweiten Pelotte an der Innenseite des Grundkörpers der Kniegelenkbandage kann diese in unterschiedlichen Therapiephasen bei der Therapie von Kniegelenkbeschwerden eingesetzt werden. Insbesondere kann in einer ersten Therapiephase eine Therapie des Kniegelenks sowohl mit der ersten als auch mit der zweiten Pelotte erfolgen, indem die zweite Pelotte an der Innenseite des Grundkörpers befestigt und die Kniegelenkbandage in diesem Zustand an einem Kniegelenk angelegt wird. In dieser ersten Therapiephase verhindert die erste Pelotte in Verbindung mit dem an den Verankerungspunkten verankerten Zugband ein Hochschieben der Patella durch die distal der Patella angelegte und quer über die Patellasehne verlaufende zweite Pelotte bei einer Bewegung des Kniegelenks, insbesondere bei einer Beugung des Knies.

In einer zweiten Therapiephase kann die zweite Pelotte von der Kniegelenkbandage gelöst und in Verbindung mit dem Halteband am Kniegelenk unterhalb (also distal) der Patella angeordnet werden. In dieser zweiten Therapiephase wirkt die zweite Pelotte zusammen mit dem Halteband als Patellasehnenbandage, welche einen Druck auf die Patellesehne ausübt und dadurch zu einer Vorspannung der Patellasehne führt, wodurch Patellasehnenschmerzen verringert werden können. Die zweite Therapiephase kann dabei auch eine präventive Therapiephase sein, die nach einer erfolgreichen ersten Therapiephase erfolgt, um ein Wiederauftreten der Kniegelenkbeschwerden zu verhindern. Insbesondere kann die zweite Therapiephase bei sportlicher Betätigung des Patienten erfolgen, um dabei das Kniegelenk zu stabilisieren und zu entlasten, wodurch Verletzungen oder Überbeanspruchungen verhindert werden können.
Um sowohl die erste als auch die zweite Therapiephase durchführen zu können, ist ein Bandagenset vorteilhaft, das eine erfindungsgemäße Bandage und ein Halteband umfasst, an dem die zweite Pelotte, insbesondere über einen Klettverschluss, lösbar befestigt werden kann. Ein solches Bandagenset ermöglicht es, die zweite Pelotte entweder zusammen mit der Kniegelenkbandage oder auch einzeln als Patellasehnenbandage (unabhängig von der Kniegelenkbandage) zu verwenden, indem die zweite Pelotte mit dem Halteband am Knie eines Patienten angelegt wird. Gegenstand der Erfindung ist daher auch ein Bandagenset, das eine Bandage sowie ein Halteband enthält, wobei die Bandage einen schlauchförmigen Grundkörper aus einem elastischen Textilmaterial mit einer Innenseite, zumindest eine am Grundkörper angeordnete erste Pelotte, welche bei angelegter Bandage proximal zur Patella positioniert ist und die Patella zumindest teilweise umgreift, sowie eine distal zur ersten Pelotte anordenbare zweite Pelotte, welche bei angelegter Bandage über die Patellasehne verläuft, umfasst, wobei die zweite Pelotte abnehmbar an der Innenseite des Grundkörpers befestigbar ist und auch an dem Halteband lösbar befestigbar ist.

In einer bevorzugten Ausführungsform der erfindungsgemäßen Kniegelenkbandage ist die erste Pelotte in dem schlauchförmigen Grundkörper integriert und insbesondere in einer Tasche angeordnet. Dadurch ist eine sichere und positionsgerechte Anordnung der ersten Pelotte um die Patella beim Anlegen der Kniegelenkbandage gewährleistet. Die Tasche ist bevorzugt an der Innenseite des Grundkörpers befestigt, beispielsweise durch Vernähen oder Verstricken mit dem Grundkörper.

In einer bevorzugten Ausführungsform ist mit der ersten Pelotte ein Zugband verbunden, wobei das Zugband ein erstes und ein zweites Ende aufweist, und des Zugband nahe oder an seinen beiden Enden distal der Patella am Grundkörper mittels lösbarer Fixiermittel an vorgegebenen Verankerungspunkten verankerbar ist. Dabei umgreift die bevorzugt U- oder hufeisenförmig ausgebildete erste Pelotte bei an einem Kniegelenk angelegter Kniegelenkbandage die Patella zumindest teilweise in ihrem proximalen Bereich und bevorzugt auch seitlich, d.h. in ihrem medialen und lateralen Bereich. Bei einer Bewegung des Kniegelenks wird dabei auf die Patella aktiv eine stabilisierende und die Patella zentrierende Kraft sowohl in medialer als auch in distaler Richtung augeübt. Das erste Ende des Zugbands ist dabei zweckmäßig an einem vorgegebenen ersten Verankerungspunkt und das zweite Ende des Zugbands an einem vorgegebenen zweiten Verankerungspunkt am Grundkörper der Bandage verankerbar, wobei bei angelegter Bandage beide Verankerungspunkte jeweils distal der Patella liegen und bevorzugt gleichzeitig entweder beide Verankerungspunkte medial zur Patellasehne liegen oder einer der beiden Verankerungspunkte medial zur Patellasehne und der andere Verankerungspunkt distal zur Patellasehne in der Linie der Verlängerung der Patellasehne in Längsrichtung auf dem Schienbein liegt.

Dadurch liegen die Verankerungspunkte der beiden Enden des Zugbands einerseits distal der Patella und andererseits entweder beide medial zur Patellasehne oder es liegt einer der beiden Verankerungspunkte medial zur Patellasehne während der andere Verankerungspunkt distal zur Patellasehne in der Linie der Verlängerung der Patellasehne in Längsrichtung auf dem Schienbein liegt. In Bezug auf eine durch das Kniegelenk verlaufende Sagittalebene liegen die beiden Verankerungspunkte der beiden Enden des Zugbands bei dieser Ausführungsform der Erfindung nicht symmetrisch um die Sagittalebene, sondern es liegen entweder beide Verankerungspunkte distal dieser Sagittalebene oder es liegt nur einer der beiden Verankerungspunkte medial zur Sagittalebene und der andere Verankerungspunkt liegt distal zur Patellasehne in dieser Sagittalebene, die durch die Verlängerung der Patellasehne in deren Längsrichtung auf dem Schienbein definiert ist.

Durch diese Anordnung der Verankerungspunkte, an denen die beiden Enden des Zugbands am Grundkörper der Kniegelenkbandage verankert werden, wird bei dieser bevorzugten Ausführungsform der erfindungsgemäßen Kniegelenkbandage bei einer Bewegung des Kniegelenks, insbesondere beim Beugen des Knies, durch die erste Pelotte, die zugfest mit dem Zugband verbunden ist, aktiv eine Kraft auf die Patella ausgeübt, die einerseits in medialer und andererseits in distaler Richtung auf die Patella einwirkt und dadurch verhindert, dass die Patella nach außen oder nach oben in eine physiologisch ungünstige Stellung verschoben wird. Diese Ausführungsform der Kniegelenkbandage bewirkt daher einerseits eine Lateralisierung der Patella und wirkt andererseits einem Hochschieben der Patella oder einem Patellahochstand entgegen.

Dieser Effekt wird dabei durch ein Zusammenwirken der aus einem elastischen Material, wie z.B. Silikon, gefertigten ersten Pelotte und der zugfest mit dieser Pelotte verbundenen Zugband sowie dessen Verankerung an den definierten Verankerungspunkten (erster und zweiter Verankerungspunkt) bewirkt.

Das Zugband ist dabei zweckmäßig unelastisch oder zumindest deutlich weniger elastisch als das Material der ersten Pelotte. Das Zugband kann entweder durchgehend vom ersten Ende zum zweiten Ende ausgebildet sein und abschnittsweise zugfest mit der ersten Pelotte verbunden sein. Insbesondere kann das durchgehend ausgebildete Zugband im Bereich der ersten Pelotte durch das elastische Material der ersten Pelotte durchlaufen und dabei mit der ersten Pelotte verbunden sein, indem das Zugband in das elastische Material der ersten Pelotte, bei dem es sich beispielsweise um Silikon oder Silikonkautschuk handeln kann, eingegossen ist. Das Zugband kann jedoch auch abschnittsweise an Unterbrechungsstellen unterbrochen sein, wobei das abschnittsweise unterbrochene Zugband an den Unterbrechungsstellen mit der ersten Pelotte zugfest verbunden ist, indem das Zugband an den Unterbrechungsstellen beispielsweise mit dem elastischen Material der ersten Pelotte verklebt ist.

In einem bevorzugten Ausführungsbeispiel der Erfindung sind die Enden des Zugbands mittels Fixiermittel an der Außenseite des Grundkörpers verankerbar. Zweckmäßig umfassen die Fixiermittel dabei lösbare Befestigungsmittel, wie z.B. Klettverschlüsse, wobei bevorzugt an jedem Ende des Zugbands ein solches Befestigungsmittel angeordnet ist, mit dem das jeweiligen Ende des Zugbands lösbar an der Außenseite des Grundkörpers befestigt werden kann. Wenn die (freien) Enden des Zugbands mittels der Befestigungsmittel an der Außenseite des Grundkörpers befestigt sind, sind die beiden Verankerungspunkte, also der erste Verankerungspunkt des ersten Endes und der zweite Verankerungspunkt des zweiten Endes, durch die Position der Befestigungsmittel definiert, über welche das jeweilige Ende des Zugbands an der Außenseite des Grundkörpers befestigt ist.

In einer weiteren Ausführungsform der Erfindung umfassen die Fixiermittel für jedes Ende des Zugbands Umlenkmittel, welche das Zugband nahe des jeweiligen Endes umlenken. Wie bei dem obigen Ausführungsbeispiel sind auch bei diesem Ausführungsbeispiel an jedem Ende des Zugbands Befestigungsmittel angeordnet, mit dem das jeweilige Ende des Zugbands lösbar an einem Abschnitt des Zugbands befestigbar ist. Dieses Ausführungsbeispiel ermöglicht die Ausübung höherer Zugkräfte aufgrund eines durch die Umlenkung des Zugbands an seinen Enden bewirkten Flaschenzugeffekts. Bei diesem Ausführungsbeispiel sind die beiden Verankerungspunkte, also der erste Verankerungspunkt des ersten Endes des Zugbands und der zweite Verankerungspunkt des zweiten Endes des Zugbands, durch die Stellen definiert, an denen das Zugband im Bereich des ersten Endes und im Bereich des zweiten Endes durch die Umlenkmittel umgelenkt wird. Die Position der Umlenkmittel, bei denen es sich beispielsweise um Umlenkösen oder -öffnungen in einer Umlenklasche handeln kann, definiert dabei die beiden Verankerungspunkte.

Die vom Zugband auf die erste Pelotte bei einer Bewegung des Kniegelenks aktiv ausgeübte Kraft kann dabei durch die Positionen bestimmt werden, an denen die beiden Enden des Zugbands entweder an der Außenseite des Grundkörpers oder - bei umgelegten Bandabschnitten - auf einem Abschnitt des jeweiligen Bands mittels der Befestigungsmittel befestigt werden. Dadurch können unterschiedlich hohe Kräfte ausgeübt werden, je nach Position der fixierten Enden des Zugbands. Die ausgewählte Zugkraft kann dabei je nach Art oder Ziel der Therapie oder dem Grad der zu therapierenden Beschwerden angepasst werden. Die Richtung der ausgeübten Kräfte wird durch die Lage des Zugbands, insbesondere der äußeren Abschnitte des Zugbands, bestimmt.

Im Bereich der Tasche, in der die erste Pelotte in einem bevorzugten Ausführungsbeispiel der Erfindung angeordnet ist, sind zweckmäßig zwei Öffnungen in dem Grundkörper vorgesehen. Die Öffnungen können insbesondere als schmale Schlitze ausgebildet sein, die sich in lateral-medialer Richtung (also etwa horizontal) erstrecken und/oder schräg zur lateral-medialer Richtung verlaufen können.

Das Zugband kann dabei aus mehreren Abschnitten zusammengesetzt sein und insbesondere einen ersten und einen zweiten Abschnitt umfassen, wobei der erste Abschnitt das erste Ende des Zugbands und der zweite Abschnitt das zweite Ende des Zugbands enthält und der erste und der zweite Abschnitt des Zugbands auf der Außenseite des schlauchförmigen Grundkörpers angeordnet sind. Der erste und der zweite Abschnitt des Zugbands sind dabei durch einen im Inneren des schlauchförmigen Grundkörpers und insbesondere durch die erste Pelotte verlaufenden dritten Abschnitt miteinander verbunden, so dass sich ein durchgehendes Zugband aus den drei Abschnitten zusammensetzt, welches bereichsweise innerhalb und bereichsweise außerhalb des schlauchförmigen Grundkörpers verläuft und durch die Öffnungen in dem Grundkörper von innen nach außen geführt ist. Der erste und der zweite Abschnitt des Zugbands liegt dadurch auf der Außenseite des schlauchförmigen Grundkörpers, weshalb der erste und der zweite Abschnitt im Folgenden auch als äußere Abschnitte bezeichnet werden.

Die Position der Öffnungen, durch die die äußeren Abschnitte des Zugbands nach außen geführt sind, bestimmt dabei zusammen mit der Position der Verankerungspunkte die Lage der äußeren Abschnitte des Zugbands, die sich jeweils zwischen der zugeordneten Öffnung und dem jeweiligen Verankerungspunkt (erster bzw. zweiter Verankerungspunkt) erstrecken.

Durch die gewählte Anordnung der Verankerungspunkte verläuft einer der beiden äußeren Abschnitte (zweiter Abschnitt) dabei zumindest im Wesentlichen in distal-proximaler Richtung, während der andere äußere Abschnitt (erster Abschnitt) schräg dazu verläuft, insbesondere von proximal-lateral nach distal-medial. In einer bevorzugten Ausführungsform schließen die beiden äußeren Abschnitte (erster und zweiter Abschnitt) des Zugbands einen Winkel im Bereich von 30° bis 60°, insbesondere von 45° ein. Durch diese Anordnung der beiden äußeren Abschnitte (erster und zweiter Abschnitt) des Zugbands erfolgt bei einer Bewegung des Knies eine Kraftausübung auf die Patella mit einer Kraftrichtung, die einerseits eine Komponente in distaler Richtung und andererseits eine Komponente in medialer Richtung aufweist.

Zur Erzielung eines Massageeffekts, der insbesondere eine durchblutungsfördernde Wirkung erzeugt, kann die erste Pelotte und die zweite über Stimulationselemente verfügen, z.B. in Form von Noppen oder Rippen, welche eine massierende Wirkung auf das die Patella und die Patellasehne umgebende Gewebe ausüben. Die erste Pelotte kann ferner eine im medial-proximalen Bereich angeordnete Ausbuchtung aufweisen, die eine stimulierende Wirkung auf die Muskulatur ausübt.

Das Textilmaterial des Grundkörpers ist elastisch ausgebildet. Insbesondere kann der Grundkörper aus einem elastischen Gestrick gebildet sein. Durch die Elastizität des schlauchförmigen Grundkörpers wird bei angelegter Kniegelenkbandage eine Kompression auf die das Kniegelenk umgebende Muskulatur ausgeübt, wodurch ein Massageeffekt und eine durchblutungsfördernde Wirkung erzeugt wird. Durch die von dem elastischen Textilmaterial des Grundkörpers ausgeübte Kompression werden weiterhin Schwellungen und Ergüsse im Bereich des Kniegelenks abgebaut.

Diese und weitere Vorteile sowie Merkmale der Erfindung ergeben sich aus dem nachfolgend unter Bezugnahme auf die begleitenden Zeichnungen näher beschriebenen Ausführungsbeispiel, wobei die Zeichnungen zeigen:
- **Fig. 1**: Vorderansicht auf eine Ausführungsform einer erfindungsgemäßen Kniegelenkbandage;
- **Fig. 2**: perspektivische Ansicht auf die Kniegelenkbandage von Figur 1 in einem an einem Kniegelenk angelegten Zustand;
- **Fig. 3**: Darstellung der Verwendung der Kniegelenkbandage von Figur 1 in einer ersten Therapiephase durch eine Schnittdarstellung der Kniegelenkbandage in einem an einem gebeugten Kniegelenk angelegten Zustand;
- **Fig. 4**: Detailansicht der zweiten Pelotte der Kniegelenkbandage von Figur 1 mit einem zugehörigen Halteband zur Ausbildung einer Patellasehnenbandage zum Anlegen der zweiten Pelotte an einem Kniegelenk;
- **Fig. 5**: Darstellung der Verwendung der Patellasehnenbandage von Figur 4 in einer zweiten Therapiephase durch eine Schnittdarstellung der Patellasehnenbandage in einem an einem gebeugten Kniegelenk angelegten Zustand, wobei die zweite Pelotte mit dem zugehörigen Halteband am Kniegelenk unterhalb der Patella angelegt ist.

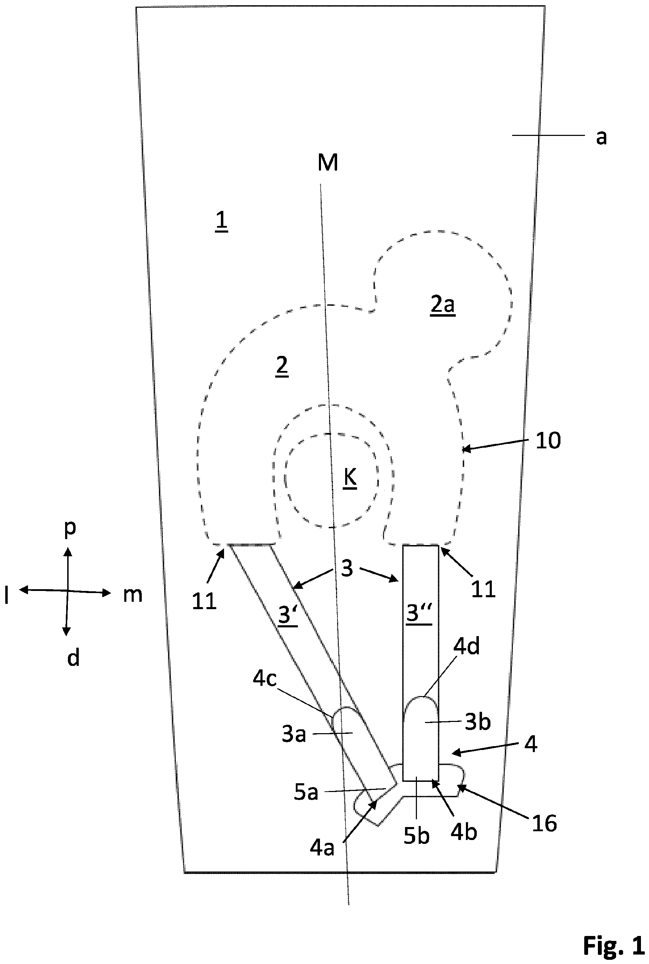

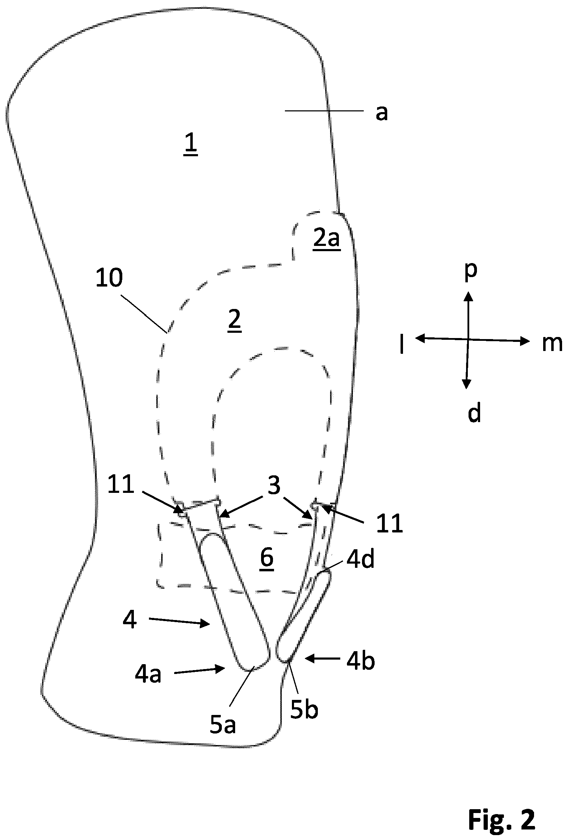

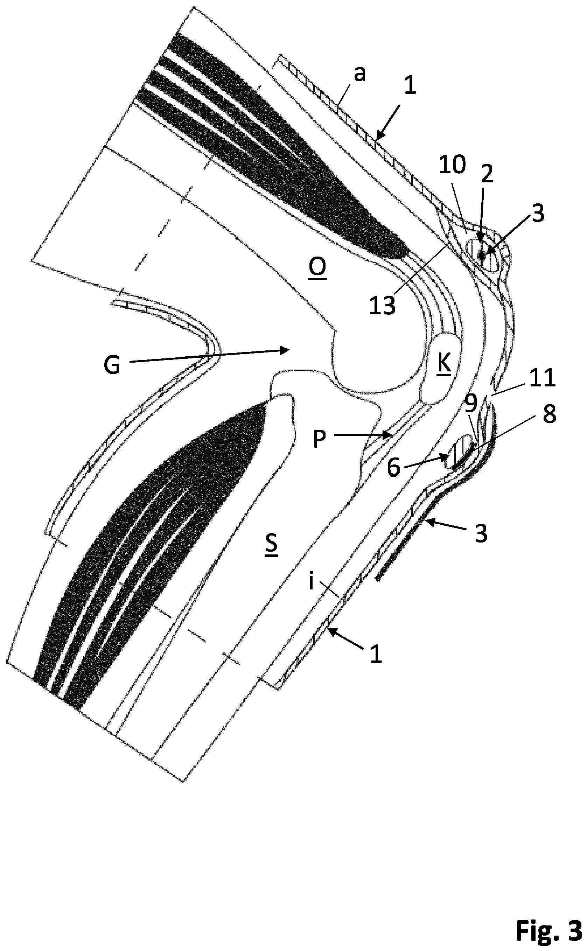

Die in den Figuren 1 und 2 gezeigte Ausführungsform einer erfindungsgemäßen Kniegelenkbandage umfasst einen schlauchförmigen Grundkörper 1 aus einem elastischen Textilmaterial, beispielsweise aus einem elastischen Gestrick, insbesondere aus einem Kompressionsgestrick, welches über einen kompressionsgebenden, elastischen Schussfaden verfügt. Der Grundkörper 1 weist dabei eine Außenseite a und bei an einem Kniegelenk G angelegter Bandage dem Kniegelenk G zugewandten Innenseite i auf, wie in Figur 3 gezeigt. Die Kniegelenkbandage umfasst weiterhin eine am Grundkörper 1 angeordnete und insbesondere in dem Grundkörper 1 integrierte erste Pelotte 2 und ein mit der ersten Pelotte 2 verbundenes Zugband 3. Die erste Pelotte 2 ist aus einem elastischen Material, beispielsweise aus Silikon oder Silikonkautschuk oder einem Gummimaterial gefertigt und ist zumindest im Wesentlichen U-förmig oder hufeisenförmig ausgebildet. Das zugfest mit der ersten Pelotte 2 verbundene Zugband ist aus einem unelastischen Material oder aus einem Material, das zumindest weniger elastisch als das Material der ersten Pelotte 2 ist und weist ein erstes Ende 3a und ein zweites Ende 3b auf. Das Zugband 3 setzt sich aus insgesamt drei Abschnitten 3', 3" und 3'" zusammen, wobei ein erster Abschnitt 3' und ein zweiter Abschnitt 3" jeweils auf der Außenseite a des Grundkörpers 1 und ein den ersten und dem zweiten Abschnitt verbindender dritter Abschnitt 3'" im Inneren des schlauchförmigen Grundkörpers 1, also an dessen Innenseite i angeordnet ist. Zum Ein- bzw. Herausführen des Zugbands 3 in das Innere des schlauchförmigen Grundkörpers 1 bzw. aus diesem heraus sind in dem Grundkörper 1 zwei Öffnungen 11, insbesondere in Form von Schlitzen vorgesehen, durch die das Zugband 3 geführt ist. Der im Inneren des schlauchförmigen Grundkörpers 1 verlaufende dritte Abschnitt 3"" des Zugbands 3 verläuft dabei innerhalb der ersten Pelotte 2. Zweckmäßig ist der dritte Abschnitt 3''' des Zugbands 3 in das elastische Material der ersten Pelotte 2 eingegossen und dadurch zugfest mit der ersten Pelotte 2 verbunden. Der innere, dritte Abschnitt 3''' des Zugbands 3 kann jedoch auch außerhalb der ersten Pelotte 2 angeordnet und mit dieser z.B. durch Verkleben zugfest verbunden sein.

Zur Aufnahme der ersten Pelotte 2 und des darin verlaufenden dritten Abschnitts 3''' des Zugbands 3 ist an der Innenseite i des Grundkörpers 1 eine in den Figuren 1 und 2 gestrichelt dargestellte Tasche 10 vorgesehen. Die Tasche 10 kann beispielsweise an der Innenseite i des Grundkörpers 1 vernäht und auf diese Weise mit dem Grundkörper 1 verbunden sein. Um einen Zugang zur Tasche 10 zu bilden, ist die Tasche 10 mit den Öffnungen 11 im Grundkörper 1 verbunden, so dass der dritte Abschnitt 3''' des Zugbands 3 durch eine der beiden Öffnungen 11 auf der einen Seite in die Tasche 10 ein- und auf der anderen Seite durch die andere Öffnung 11 aus der Tasche 10 herausgeführt werden kann.

Wie insbesondere aus der Vorderansicht der Figur 1 ersichtlich weist die Tasche 10 und die darin angeordnete erste Pelotte 2 eine Ausbuchtung 2a im medial-proximalen Bereich auf. Diese Ausbuchtung 2a dient dazu eine stimulierende Wirkung auf die Muskulatur auszuüben.. Wie weiterhin insbesondere der Vorderansicht der Figur 1 zu entnehmen ist, umgreift die in der Tasche 10 angeordnete erste Pelotte 2 die in Figur 1 gestrichelt dargestellte Patella K im proximalen sowie im medialen und lateralen Bereich. In den Figuren 1 und 2 sind die auf das Kniegelenk G bezogenen Richtungen medial m, lateral 1, proximal p und distal d durch Pfeile angegeben.

Die beiden äußeren Abschnitte 3' und 3" des Zugbands 3 sind mittels Fixiermittel 4 festgelegt. Bei dem in den Figuren 1 und 2 gezeigten Ausführungsbeispiel ist sowohl der erste Abschnitt 3', der das erste Ende 3a des Zugbands 3 enthält, und der zweite Abschnitt 3", der das zweite Ende 3b des Zugbands 3 enthält, über jeweils ein zugeordnetes Umlenkmittel 4a, 4b um 180° umgelenkt. Dabei ist sowohl am ersten Ende 3a als auch am zweiten Ende 3b des Zugbands 3 jeweils an Befestigungsmittel 4c, 4d angeordnet, mit dem der um 180° umgelenkte Abschnitt des Zugbands 3 an dem jeweiligen äußeren Abschnitt 3' bzw. 3" fixiert ist. Bei den Umlenkmitteln 4a, 4b kann es sich beispielsweise um Umlenkösen oder - wie in den Ausführungsbeispielen der Figuren 1 und 2 gezeigt - um Umlenköffnungen in einer auf der Außenseite a des Grundkörpers 1 befestigten Lasche 16 handeln.

Die Position der Umlenkmittel 4a, 4b definiert dabei für jeden der beiden Abschnitte 3' und 3" des Zugbands 3 einen Verankerungspunkt, an dem der jeweilige Abschnitt 3' bzw. 3" des Zugbands 3 in Bezug auf den Grundkörper 1 verankert ist. So definiert das dem ersten Abschnitt 3' des Zugbands 3 zugeordnete erste Umlenkmittel 4a einen ersten Verankerungspunkt 5a und das dem zweiten Abschnitt 3" zugeordnete zweite Umlenkmittel 4b definiert einen zweiten Verankerungspunkt 5b. Wie insbesondere aus der Vorderansicht der Figur 1 ersichtlich, liegen sowohl der erste Verankerungspunkt 5a als auch der zweite Verankerungspunkt 5b sowohl distal als auch medial zu der Sagittalebene M, die durch den Mittelpunkt der Patella K geht und die Mittellängsachse der Patellasehne P enthält. Die unterhalb der Patella K sich erstreckende Patellasehne P, welche die Patella K mit dem Schienbein S verbindet, erstreckt sich dabei in dieser Sagittalebene M. Damit liegen die beiden Verankerungspunkte 5a, 5b sowohl medial als auch distal zur Patellasehne P.

Die Position der Verankerungspunkte bestimmt dabei zusammen mit der Position der Öffnungen 11, durch die die äußeren Abschnitte 3', 3'' des Zugbands 3 nach außen geführt sind, die Lage der äußeren Abschnitte 3' und 3" in Bezug auf den Grundkörper 1, wobei sich die sich die äußeren Abschnitte 3' und 3" jeweils zwischen der zugeordneten Öffnung 11 und dem ersten Verankerungspunkt 5a bzw. dem zweiten Verankerungspunkt 5b erstrecken.

Durch die Anordnung der Verankerungspunkte 5a, 5b des Zugbands 3 kann bei einer Festlegung des Zugbands 3 mittels der Befestigungsmittel 4c, 4d eine Zugkraft auf die zugfest mit dem Zugband 3 verbundene erste Pelotte 2 ausgeübt werden, wobei diese Zugkraft im Wesentlichen in distaler Richtung gerichtet ist und aufgrund der asymmetrischen Anordnung der beiden Verankerungspunkte 5a, 5 b bezüglich der Sagitallebene M bzw. in Bezug auf die Patellesehne P auch eine Kraftkomponente in medialer Richtung aufweist. Die Richtungen der von den beiden äußeren Abschnitten 3' und 3" des Zugbands 3 auf die erste Pelotte 2 ausgeübten Zugkräfte ergeben sich durch die Lage der beiden äußeren Abschnitte 3' und 3" des Zugbands 3, die dem Ausführungsbeispiel der Figur 1 zu entnehmen ist. In diesem Ausführungsbeispiel schließen die beiden äußeren Abschnitte 3' und 3" des Zugbands 3 miteinander einen Winkel von ca. 45° ein.

Bei einer Bewegung des Kniegelenks, insbesondere bei einer Beugung des Knies, werden die beiden Verankerungspunkte 5a, 5b, an denen das Zugband 3 auf der Außenseite a des Grundkörpers 1 verankert ist, in distaler Richtung nach unten bewegt, wodurch die beiden außenliegenden Abschnitte 3' und 3" des Zugbands 3 eine in der jeweiligen Längsrichtung des ersten bzw. des zweiten Abschnitts 3', 3" gerichtete Zugkraft auf die erste Pelotte 2 ausüben.

Durch den in distal-proximaler Richtung verlaufenden zweiten Abschnitt 3" des Zugbands 3 wird auf die erste Pelotte 2 eine Kraft in distaler Richtung ausgeübt. Aufgrund der asymmetrischen Anordnung der beiden Verankerungspunkte 5a, 5b in Bezug auf die Sagitallebene M bzw. in Bezug auf die Patellasehne P und die damit verbundene Schrägstellung des ersten Abschnitts 3' des Zugbands 3 (wie aus Figur 1 ersichtlich) wirkt auf die erste Pelotte 2 weiterhin eine durch den ersten Abschnitt 3' des Zugbands 3 ausgeübte Zugkraft, welche eine Kraftkomponente in distaler Richtung und eine Kraftkomponente in medialer Richtung umfasst. Insgesamt wirkt durch das Zugband 3 auf die erste Pelotte 2 damit eine in distaler und medialer Richtung weisende Kraft ein, wodurch bei einer Bewegung des Kniegelenks, insbesondere bei einer Beugung des Knies, eine Zentrierung der Patella K in ihre physiologisch korrekte Stellung erfolgt, indem die vom Zugband 3 auf die erste Pelotte 2 übertragene Zugkraft in distaler und medialer Richtung auf die Patella K einwirkt, welche in ihrem proximalen sowie medial-lateralen Bereich von der ersten Pelotte 2 umgeben ist. Dadurch erfolgt bei einer Bewegung des Kniegelenks durch die vom Zugband 3 auf die erste Pelotte 2 ausgebübte Zugkraft einerseits eine Lateralisierung der Patella K und andererseits wird ein Hochschieben der Patella K in proximaler Richtung unterdrückt.

Dieser Mechanismus ist in der Schnittdarstellung der Figur 3 zu erkennen, in der die Verwendung der Kniegelenkbandage in einer ersten Therapiephase durch eine Schnittdarstellung der Kniegelenkbandage in einem an einem gebeugten Kniegelenk angelegten Zustand gezeigt ist. Dabei ist die Kniegelenkbandage so am Kniegelenk G angelegt, dass die erste Pelotte 2 mit dem darin verlaufenden dritten Abschnitt 3''' des Zugbands 3 die Patella K in ihrem proximalen sowie im lateralen und medialen Bereich umgreift.

In der Anwendungsform der erfindungsgemäßen Kniegelenkbandage, welche in der Schnittdarstellung der Figur 3 gezeigt ist, umfasst die Kniegelenkbandage neben der ersten Pelotte 2, die an der Innenseite i des Grundkörpers 1 in der Tasche 10 angeordnet ist, noch eine zweite Pelotte 6, welche ebenfalls an der Innenseite i des Grundkörpers 1 befestigt und distal zur Patella K angeordnet ist. In der Funktionsdarstellung der Figur 2 ist die Lage der zweiten Pelotte 6 im Bereich unterhalb der ersten Pelotte 2 gestrichelt dargestellt. Die zweite Pelotte 6 ist dabei bandförmig ausgebildet und verläuft bei am Kniegelenk G angelegter Kniegelenkbandage quer über die Patellasehne P, wie aus Figur 3 ersichtlich.

Die dadurch als Patellasehnenbandage wirkende zweite Pelotte 6 ist dabei erfindungsgemäß abnehmbar an der Innenseite i des Grundkörpers 1 befestigt, beispielsweise durch zusammenwirkende Befestigungselemente 8, 9. Bei den Befestigungselementen 8, 9 kann es sich beispielsweise um die Elemente eines Klettverschlusses, insbesondere um ein Hakenteil und ein zugehöriges Flauschteils eines Klettverschlusses handelt. Bevorzugt ist an der dem Kniegelenk G abgewandten Außenseite der zweiten Pelotte 6 ein Befestigungselement 8 in Form eines Hakenteils eines Klettverschlusses angeordnet, welches mit einem dazu korrespondierenden Flauschteil an der Innenseite i des Grundkörpers 1 zusammenwirkt, um die bandförmige zweite Pelotte 6 abnehmbar an der Innenseite i des Grundkörpers 1 zu befestigen.

Wenn die zweite Pelotte 6 an der aus Figur 3 ersichtlichen Position an der Innenseite i des Grundkörpers 1 befestigt ist, übt die zweite Pelotte 6 einen Druck auf die Patellasehne P aus und setzt diese damit bei einer Bewegung des Kniegelenks unter Spannung. Dadurch werden die Sehnen, Knochenansätze und Knorpelstrukturen im Kniegelenk entlastet. Die zweite Pelotte 6 wirkt dadurch stabilisierend und entlastend auf das Kniegelenk und wirkt Bewegungseinschränkungen bei einer Degeneration des Kniegelenks entgegen.

Gleichzeitig wird bei einer Bewegung des Kniegelenks G, insbesondere beim Beugen des Knies, ein Hochschieben der Patella K in proximaler Richtung, welches durch den von der zweiten Pelotte 6 ausgeübten Druck auf die Patellasehne P verursacht werden kann, durch die proximal der Patella K angeordnete und diese im proximalen sowie im medial-lateralen Bereich umgebende erste Pelotte 2 verhindert. Damit wird die Patella K bei angelegter Kniegelenkbandage bei einer Beugung des Knies in ihrer physiologisch korrekten Lage zentriert, ohne dass es zu einem Hochschieben der Patella K in proximaler Richtung (nach oben) und zu einem Abdriften in lateraler Richtung (nach außen) kommen kann.

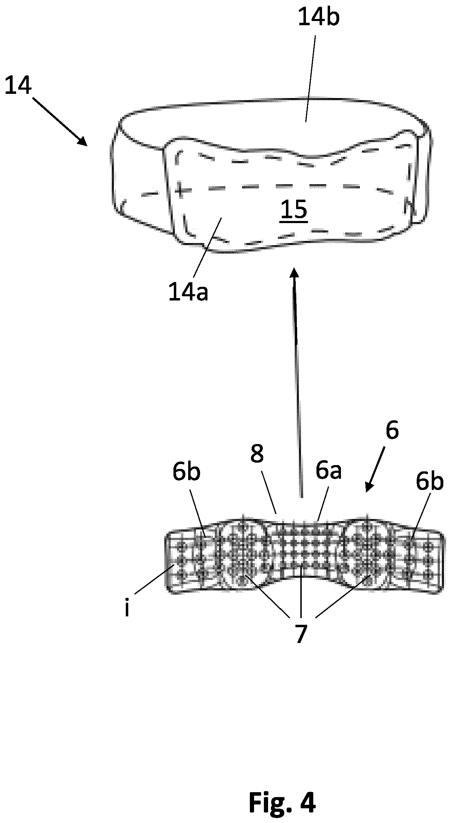

Die in Figur 4 unten im Detail gezeigte zweite Pelotte 6 ist zumindest im Wesentlichen bandförmig ausgebildet und weist eine Längsseite auf, die sich bei angelegter Kniegelenkbandage quer über die Patellasehne P erstreckt, wenn die zweite Pelotte 6, wie in Figur 3 gezeigt, an der Innenseite i des Grundkörpers 1 befestigt ist. Die zweite Pelotte 6 weist dabei zweckmäßig einen mittleren Abschnitt 6a sowie zwei sich seitlich daran anschließende randseitige Abschnitte 6b auf, wobei die randseitigen Abschnitte 6b vom mittleren Abschnitt 6a nach unten, d.h., in distaler Richtung, abgekröpft sind, wie aus Figur 4 unten ersichtlich. Unter abgekröpft wird dabei verstanden, dass die randseitigen Abschnitte 6b winklig unter einem Winkel im Bereich von 10° bis 30° zum mittleren Abschnitt 6a verlaufen. Die distal abgekröpften randseitigen Abschnitte 6b ermöglichen eine bessere Passform und Anschmiegsamkeit über die Hoffa'sehen Fettkörper.

Zur Erzielung eines Massageeffekts bei angelegter zweiter Pelotte 6 weist die bei angelegter Kniebandage dem Kniegelenk zugewandte Innenseite der zweiten Pelotte 6 eine Mehrzahl von Stimulationselementen 7 auf, insbesondere in Form von Erhebungen oder Vorsprüngen und bevorzugt in Form von Noppen.

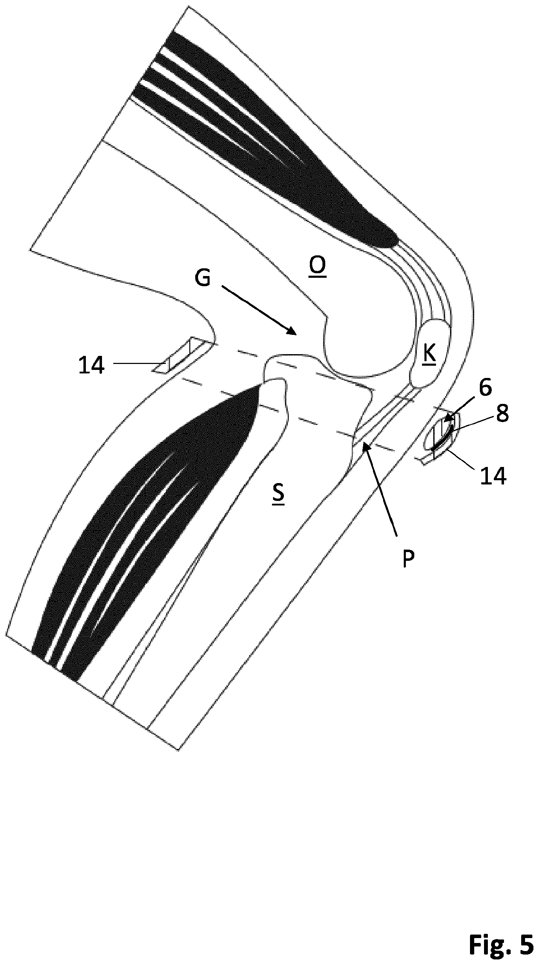

Die in Figur 4 unten gezeigte zweite Pelotte 6 kann in einer gesonderten zweiten Therapiephase auch ohne den Grundkörper 1 mit der darin integrierten ersten Pelotte 2 verwendet werden. Hierzu ist ein in Figur 4 oben gezeigtes Halteband 14 vorgesehen, welches über einen Aufnahmeabschnitt 14a verfügt, an dem die zweite Pelotte 6 abnehmbar befestigt werden kann. Zweckmäßig weist der Aufnahmeabschnitt 14a lösbare Befestigungsmittel 15 auf, insbesondere einen Teil eines Klettverschlusses, mit denen die zweite Pelotte 6 am Aufnahmeabschnitt 14a des Haltebands 14 befestigt werden kann. Wenn auf der einen Seite (Außenseite) der zweiten Pelotte 6 ein Hakenteil eines Klettverschlusses angeordnet ist, mit dem die zweite Pelotte 6 an der Innenseite i des Grundkörpers 1 befestigbar ist (wie in Figur 3 gezeigt), sind die Befestigungsmittel 15 am Aufnahmeabschnitt 14a des Haltebands 14 zweckmäßig als dazu korrespondierendes Flauschteil eines Klettverschlusses ausgebildet. Zur Befestigung des Haltebands 14 am Kniegelenk G weist das Halteband 14 einen mit dem Aufnahmeabschnitt 14a verbundenen Halteabschnitt 14b auf, wobei der Halteabschnitt 14b bevorzugt als längenverstellbarer Gurt oder als elastisches Band, beispielsweise als Gummiband, ausgebildet ist. Über den längenverstellbaren bzw. elastischen Halteabschnitt 14b kann das Halteband 14 mit der am Aufnahmeabschnitt 14a befestigten zweiten Pelotte 6 am Kniegelenk G angelegt werden, wie in Figur 5 gezeigt. Dabei verläuft das Halteband 14 im Bereich unterhalb der Kniekehle und erstreckt sich über den Umfang des oberen Abschnitts des Unterschenkels, wobei die an der Innenseite am Aufnahmeabschnitt 14a des Haltebands 14 befestigte zweite Pelotte 6 im Bereich der Patellasehne P liegt und sich quer über die Patellasehne P erstreckt. In dieser Position der zweiten Pelotte 6 übt diese insbesondere bei einer Bewegung des Kniegelenks G einen Druck auf die Patellasehne P aus. Durch die Stimulationselemente 7 wird die Patellasehne P und das diese umgebende Gewebe stimuliert, wodurch die Durchblutung des Körpergewebes verbessert wird.

Die erfindungsgemäße Kniegelenkbandage kann damit einerseits in der in Figur 3 gezeigten Weise und andererseits in der in Figur 5 gezeigten Weise zur Therapie von Kniegelenksbeschwerden verwendet werden, wobei in der in Figur 3 gezeigten ersten Verwendungsform sowohl die erste Pelotte 2 als auch die zweite Pelotte 6 einen Druck auf bestimmte Bereiche des Kniegelenks G ausübt und in der in Figur 5 gezeigten zweiten Verwendungsform lediglich durch die zweite Pelotte 6 ein Druck auf die Patellasehne P ausgeübt wird.

Die erfindungsgemäße Kniegelenkbandage kann daher zur Behandlung von unterschiedlichen Kniebeschwerden, wie z.B. bei Schädigungen des Kniegelenks im Gleitlager zwischen Patella und Oberschenkel, bei einem Patellahochstand, beim femoropatellaren Schmerzsyndrom, bei Patella-Luxationen oder -Subluxationen, bei Morbus Osgood Schlatter, bei einer Gonararthrose oder bei Gelenkergüssen und Reizzuständen im Kniegelenk einerseits therapierend und andererseits auch präventiv eingesetzt werden. Insbesondere kann eine kombinierte Therapie mit einer Verwendung der erfindungsgemäßen Kniegelenkbandage in der in Figur 3 gezeigten Verwendungsform in einer ersten Therapiephase und danach in einer zweiten Therapiephase in der in Figur 5 gezeigten Verwendungsform zielführend sein. In der ersten Therapiephase kann die Bandage bspw. mit an der Innenseite befestigter zweiter Pelotte 6 bei einer Therapiedauer von 10 bis 100 Tagen eingesetzt werden, um die Beschwerden bei Kniegelenksdefekten zu lindern und die Ursache zu beseitigen. An diese erste Therapiephase kann sich die zweite Therapiephase anschließen, in der die zweite Pelotte 6 zusammen mit dem zugehörigen Halteband 14 als Patellasehnenbandage wie aus Figur 3 ersichtlich am Kniegelenk angelegt wird. Die zweite Therapiephase kann dabei auch eine präventive Phase sein, in der die Patellasehnenbandage bspw. zur Prävention nur während sportlicher Betätigung des Patienten eingesetzt wird.

Die Erfindung ist nicht auf das hier zeichnerisch dargestellte Ausführungsbeispiel beschränkt. So kann beispielsweise auf die Umlenkung der beiden äußeren Abschnitte 3', 3" des Zugbands 3 verzichtet werden. Anstelle der zum Umlenken der Abschnitte 3', 3" des Zugbands 3 eingesetzten Umlenkmittel 4a, 4b können die beiden Enden 3a, 3b des Zugbands 3 auch unmittelbar ohne Umlenkung mittels der an den Enden 3a, 3b angeordneten Befestigungsmittel 4c, 4c an der Außenseite a des Grundköpers 1 befestigt werden. In diesem, hier zeichnerisch nicht dargestellten Ausführungsbeispiel der erfindungsgemäßen Kniegelenkbandage werden die Verankerungspunkte 5a, 5b des Zugbands 3 durch die Position der Befestigungsmittel 4c, 4d definiert, durch welche die Enden 3a, 3b des Zugbands 3 am Grundkörper 1 befestigt sind. Maßgeblich für die Definition der beiden Verankerungspunkte 5a, 5b ist dabei die Stelle, an der die Abschnitte 3', 3" des Zugbands 3 am Grundkörper 1 verankert oder fixiert sind.

Weiterhin können sowohl die erste Pelotte 2 als auch die zweite Pelotte 6 anders ausgeformt sein. Insbesondere können an der ersten Pelotte 2 noch weitere Ausbuchtungen oder zusätzliche Stimulationselemente vorgesehen sein, welche insbesondere zu einer interplanetaren Stimulation des Kniegelenks G beitragen.

Weiterhin kann einer der beiden Verankerungspunkte distal der Patella K liegend auch in der Linie der Verlängerung der Patellasehne P in deren Längsrichtung auf dem Schienbein S liegen. Auch in diesem Fall ist eine asymmetrische Anordnung der beiden Verankerungspunkte 5a, 5b in Bezug auf die Sagittalebene M bzw. bezüglich der Patellasehne P gegeben, die eine Kraftkomponente der vom Zugband 3 auf die erste Pelotte 3 übertragenen Zugkraft in medialer Richtung erzeugt.

## Patentansprüche

1. Bandage für ein Kniegelenk (K), umfassend einen schlauchförmigen Grundkörper (1) aus einem elastischen Textilmaterial mit einer bei am Kniegelenk (G) angelegter Bandage dem Kniegelenk (G) zugewandten Innenseite (i), zumindest eine am Grundkörper angeordnete erste Pelotte (2), welche bei angelegter Bandage proximal zur Kniescheibe (K) positioniert ist und die Kniescheibe (K) zumindest teilweise umgreift, sowie eine distal zur ersten Pelotte (2) angeordnete zweite Pelotte (6), welche bei angelegter Bandage über die Patellasehne (P) verläuft, **dadurch gekennzeichnet, dass** die zweite Pelotte (6) abnehmbar an der Innenseite des Grundkörpers (1) befestigt ist.

2. Bandage nach Anspruch 1, **dadurch gekennzeichnet, dass** die zweite Pelotte (6) an ihrer bei angelegter Bandage dem Kniegelenk (G) abgewandten Außenseite (a) Befestigungselemente (8) zur lösbaren Befestigung an der Innenseite (i) des Grundkörpers (1) aufweist.

3. Bandage nach einem der Ansprüche 1 oder 2, **dadurch gekennzeichnet, dass** die zweite Pelotte (6) an ihrer zum Kniegelenk (G) zugewandten Innenseite (i) Stimulationselemente (7), bevorzugt in Form von Erhebungen oder Vorsprüngen, insbesondere in Form von Noppen, aufweist.

4. Bandage nach einem der voranstehenden Ansprüche, **dadurch gekennzeichnet, dass** die zweite Pelotte (6) zumindest im Wesentlichen bandförmig mit einer Längsseite ausgebildet ist, wobei die zweite Pelotte (6) so an der Innenseite (i) des Grundkörpers (1) befestigt ist, dass sie bei angelegter Bandage mit ihrer Längsseite quer über die Patellasehne (P) verläuft.

5. Bandage nach einem der voranstehenden Ansprüche, **dadurch gekennzeichnet, dass** die zweite Pelotte (6) zumindest im Wesentlichen bandförmig ist und einen bei angelegter Bandage quer über die Patellasehne (P) verlaufenden mittleren Abschnitt (6a) sowie zwei sich daran anschließende randseitige Abschnitte (6b) aufweist, wobei die randseitigen Abschnitte (6b) vom mittleren Abschnitt (6a) nach distal abgekröpft sind.

6. Bandage nach einem der voranstehenden Ansprüche, **gekennzeichnet durch** ein mit der ersten Pelotte (2) verbundenes Zugband (3) mit einem ersten Ende (3a) und einem zweiten Ende (3b), wobei die erste Pelotte (2) bei angelegter Bandage proximal zur Kniescheibe (K) positioniert ist und die Kniescheibe (K) zumindest teilweise umgreift und die beiden Enden (3a, 3b) des Zugbands (3) distal der Kniescheibe am Grundkörper (1) mittels lösbarer Fixiermittel (4) fixierbar sind.

7. Bandage nach Anspruch 6, **dadurch gekennzeichnet, dass** das erste Ende (3a) des Zugbands (3) an einem vorgegebenen ersten Verankerungspunkt (5a) und das zweite Ende (3b) des Zugbands (3) an einem vorgegebenen zweiten Verankerungspunkt (5b) fixierbar ist, wobei bei angelegter Kniegelenkbandage beide Verankerungspunkte (5a, 5b) jeweils distal der Kniescheibe (K) liegen und gleichzeitig entweder beide Verankerungspunkte (5a, 5b) medial zur Patellasehne (P) liegen oder einer der beiden Verankerungspunkte (5b) medial zur Patellasehne (P) und der andere Verankerungspunkt (5a) distal zur Patellasehne (P) in der Linie der Verlängerung der Patellasehne (P) in Längsrichtung auf dem Schienbein (S) liegt.

8. Bandage nach einem der voranstehenden Ansprüche, **dadurch gekennzeichnet, dass** die erste Pelotte (2) zumindest im Wesentlichen U-förmig oder hufeisenförmig ausgebildet ist und bei angelegter Bandage zumindest den proximalen Bereich der Kniescheibe (K) umgibt.

9. Bandage nach Anspruch 8, **dadurch gekennzeichnet, dass** die erste Pelotte (2) im medial-proximalen Bereich eine Ausbuchtung (2a) mit daran angeordneten Stimulationselementen aufweist.

10. Bandage nach einem der voranstehenden Ansprüche, **dadurch gekennzeichnet, dass** an der Innenseite (i) des Grundkörpers (1) ein Befestigungsmittel (9), insbesondere ein Teil eines Klettverschlusses, zur lösbaren Befestigung der zweiten Pelotte (6) angeordnet ist.

11. Bandagenset, umfassend eine Bandage nach einem der voranstehenden Ansprüche und ein Halteband (14), an dem die zweite Pelotte (6), insbesondere über einen Klettverschluss, lösbar befestigt werden kann.

12. Bandagenset nach Anspruch 11, **dadurch gekennzeichnet, dass** das Halteband (14) längenverstellbar und/oder elastisch ausgebildet ist.

13. Bandagenset nach Anspruch 11 oder 12, **dadurch gekennzeichnet, dass** das Halteband (14) einen Aufnahmeabschnitt (14a) und einen am Aufnahmeabschnitt (14a) befestigten Halteabschnitt (14b) aufweist, wobei der Aufnahmeabschnitt (14a) lösbare Befestigungsmittel (15), insbesondere einen Teil eines Klettverschlusses, enthält, mit denen die zweite Pelotte (6) am Aufnahmeabschnitt (14a) des Haltebands (14) befestigbar ist.

14. Bandagenset nach Anspruch 13, **dadurch gekennzeichnet, dass** der Halteabschnitt (14b) des Haltebands (14) einen längenverstellbaren Gurt oder ein elastisches Band aufweist.

15. Bandagenset nach Anspruch 13 oder 14, **dadurch gekennzeichnet, dass** die Befestigungsmittel (15) am Aufnahmeabschnitt (14a) des Haltebands (14) mit den Befestigungselementen (8) an der Außenseite der zweiten Pelotte (6) zusammenwirken, um eine lösbare Befestigung der zweiten Pelotte (6) an dem Aufnahmeabschnitt (14a) des Haltebands (14) zu ermöglichen.
